(19) Europäisches Patentamt
European Patent Office
Office européen des brevets

(11) Numéro de publication: **0 005 658**
**A1**

(12) # DEMANDE DE BREVET EUROPEEN

(21) Numéro de dépôt: 79400268.3

(22) Date de dépôt: 25.04.79

(51) Int. Cl.²: C 07 C 103/52
A 61 K 37/02

(30) Priorité: 27.04.78 FR 7812543

(43) Date de publication de la demande: 28.11.79 Bulletin 79/24

(84) Etats Contractants Désignés: BE CH DE GB IT LU NL SE

(71) Demandeur: Société dite: Laboratoire le Brun S.A.
5 rue de Lübeck
F-75116 Paris(FR)

(72) Inventeur: Lecomte, Jeanne-Marie
5, Rue de Lübeck
F-75116 Paris(FR)

(72) Inventeur: Roques, Bernard
4 Avenue de l'Observatoire
F-75006 Paris(FR)

(72) Inventeur: Schwartz, Jean-Charles
2 ter d'Alésia
F-75014 Paris(FR)

(74) Mandataire: Moncheny, Michel et al,
c/o Cabinet Lavoix 2 Place d'Estienne d'Orves
F-75441 Paris Cedex 09(FR)

(54) **Nouveaux dérivés de peptides analogues des enképhalines, leur procédé de préparation et leur application thérapeutique.**

(57) La présente invention est relative à des dérivés de peptides répondant à la formule générale

Tyr - D Ala - Gly - A - B - NH - $C_nH_{2n}$ - Y ,
1 2 3 4 5

dans laquelle

*A* est Phe ou pF-Phe,

*B* est Met, Leu, monofluoro-Leu, Pro, monofluoro-Pro ou une simple liaison,

*Y* est un atome d'halogène ; un radical alkyle en $C_1$ à $C_4$ mono- ou polysubstitué par au moins un groupe choisi parmi un atome d'halogène et un groupe phényle éventuellement substitué par au moins un atome d'halogène ; un radical amino -NHX; un radical sulfamido $-NHSO_2X$ ; un radical carbonyle -COX ; un radical acylamino $-NHCO(CH_2)_mX$ dans lesquels X est un atome d'halogène: un radical alkyle en $C_1$ à $C_4$ mono- ou polysubstitué par un atome d'halogène ; phényle éventuellement mono-, di-ou trisubstitué par des radicaux choisis parmi un atome d'halogène, un groupe mono- ou polyhaloalkyle en $C_1$ à $C_4$, alcoxy en $C_1$ à $C_4$ ; benzhydryle éventuellement substitué par au moins un atome d'halogène ; α ou β naphtyle éventuellement substitué par un atome d'halogène ; un groupe alkyle en $C_1$ à $C_4$, alcoxy en $C_1$ à $C_4$ ou dialkyl ($C_1$-$C_4$) amino ; ou un reste d'un radical mono- ou polycyclique choisi parmi le thiophène, la quinoléine l'isoquinoléine l'acridine et la pyridine ;

*m* est O ou un nombre de 1 à 4, et

*n* est O ou un nombre de 1 à 6,

et leurs sels d'addition avec des acides pharmaceutiquement acceptables.

Ces dérivés ont notamment des activités analgésique psychotrope et antidiarrhéique.

EP 0 005 658 A1

Nouveaux dérivés de peptides analogues des enképhalines, leur procédé de préparation et leur application thérapeutique.

La présente invention est relative à des dérivés de polypeptides ayant de fortes activités analgésique et psychotrope, qui sont des analogues de l'enképhaline.

L'enképhaline naturelle répond à la formule générale suivante :

$$\underset{1}{\text{Tyr}} - \underset{2}{\text{Gly}} - \underset{3}{\text{Gly}} - \underset{4}{\text{Phe}} - \underset{5}{\text{W}},$$

dans laquelle W est Leu-OH ou Met-OH. Ce composé naturel est un analgésique par la voie intraencéphalique, mais il est inactif in vivo par une voie d'administration normale, ce qui complique et réduit donc ses applications thérapeutiques possibles.

L'enképhaline naturelle a déjà été modifiée en différentes positions, notamment en positions 2 et 3 dans le brevet US 4 028 319 et en positions 1,4 et 5 dans le brevet DT 2 655 149.

La présente invention a pour objet des dérivés de polypeptides de formule générale :

$$\underset{1}{\text{Tyr}} - \underset{2}{\text{D Ala}} - \underset{3}{\text{Gly}} - \underset{4}{\text{A}} - \underset{5}{\text{B}} - NH - C_nH_{2n} - Y,$$

dans laquelle :

A est Phe ou pF - Phe,

B est Met, Leu, monofluoro-Leu, Pro, monofluoro-Pro ou une simple liaison,

Y est un atome d'halogène ; un radical alkyle en $C_1$ à $C_4$ mono- ou polysubstitué par au moins un groupe choisi parmi un atome d'halogène et un groupe phényle éventuellement substitué par

au moins un atome d'halogène ; un radical amino-NHX; un radical sulfamido-$NHSO_2X$ ; un radical carbonyle-COX ; un radical acylamino-$NHCO(CH_2)_mX$, dans lesquels X est un atome d'halogène ; un radical alkyle en $C_1$ à $C_4$ mono- ou polysubstitué par un atome d'halogène ; phényle éventuellement mono-, di- ou trisubstitué par des radicaux choisis parmi un atome d'halogène, un groupe mono- ou polyhaloalkyle en $C_1$ à $C_4$, alcoxy en $C_1$ à $C_4$ ; benzhydryle éventuellement substitué par au moins un atome d'halogène; $\alpha$ ou $\beta$ naphtyle éventuellement substitué par un atome d'halogène, un groupe alkyle en $C_1$ à $C_4$, alcoxy en $C_1$ à $C_4$ ou dialdyl ($C_1$-$C_4$) amino ; ou un reste d'un radical mono- ou polycyclique choisi parmi le thiophène, la quinoléine, l'isoquinoléine, l'acridine et la pyridine ; m est 0 ou un nombre de 1 à 4, et n est 0 ou un nombre de 1 à 6, et leurs sels d'addition avec des acides pharmaceutiquement acceptables.

Les composés selon la présente invention répondant à la formule I ci-dessus, et leurs sels pharmaceutiquement acceptables ont des activités analgésiques, psychotrope et antidiarrhéique, lorsqu'ils sont administrés par une voie normale, telle que la voie parentérale, orale ou rectale.

La présente invention a également pour objet un procédé de préparation des dérivés de peptides de formule I, caractérisé en ce qu'on prépare ces peptides :

a) par une condensation fragmentaire, usuelle dans la chimie des peptides, de fragments de peptides selon le schéma de condensation 1-2 + 3-5, ou

b) par un mode de constitution progressive, dans lequel d'autres groupes fonctionnels sont bloqués intermédiairement par des groupes protecteurs éliminables par hydrolyse ou hydrogénation ou en milieu alcalin, pour obtenir un peptide de formule III.

Tyr - D Ala - Gly - A - B - OH,

dans laquelle A et B ont les définitions données ci-dessus, et

c) on condense sur le groupe carboxylique terminal du peptide obtenu de formule III, une amine de formule

$$H_2N - C_n H_{2n} - Y \qquad (II)$$

dans laquelle n et Y ont les définitions données ci-dessus.

Il est bien connu des spécialistes que divers peptides peuvent être produits en condensant un amino-acide ou un peptide partiel (c'est-à-dire un peptide ayant un nombre d'unités plus faible) avec un amino-acide ou un peptide partiel. Il existe un certain nombre de procédés ou de techniques pour la mise en oeuvre de la réaction de condensation qui sont bien établis.

Par exemple, le ou les groupes fonctionnels (c'est-à-dire les groupes amino, carboxy, hydroxy) qui ne sont pas impliqués dans la réaction de formation de la liaison peptide (c'est-à-dire -CONH) lors de la réaction de condensation peuvent être protégés par un ou des groupes protecteurs avant la réaction de condensation. Dans le procédé selon la présente invention, chaque fragment de peptide ou peptide partiel peut également être protégé en ce qui concerne le ou les groupes fonctionnels qui ne doivent pas prendre part à la réaction de condensation selon des procédés connus.

Il est bien connu qu'avant la réaction de formation de la liaison peptide, le groupe carboxylique sur le C terminal qui est impliqué dans la réaction de formation de la liaison peptide, est activé pour lui permettre de favoriser la formation de la liaison peptide et s'il n'est pas activé, la réaction de formation de la liaison peptide est effectuée en présence d'un agent déshydratant. Des procédés pour l'activation du groupe carboxylique sur le C terminal sont bien établis. Le procédé selon la présente invention peut utiliser un procédé bien connu de l'art antérieur. Ceci revient à dire que la condensation selon la présente invention peut être effectuée dans un premier stade par l'activation du groupe carboxylique sur le C terminal d'un fragment de peptide et, dans le deuxième stade, par la réaction de formation de la liaison entre un fragment activé et l'autre fragment non activé, ou bien encore effectuée par une réaction de formation de la liaison peptide entre les deux fragments non activés en présence d'un agent déshydratant, les fragments de peptides étant éventuellement protégés.

De plus, il est connu d'éliminer après la réaction de condensation le ou les groupes de protection lorsque le groupe ou les groupes fonctionnels mentionnés ci-dessus sont protégés par le ou les groupes protecteurs avant la réaction de condensation. Dans le procédé selon la présente invention, l'élimination de la protection peut également être effectuée selon des techniques connues.

Dans la condensation fragmentaire selon a), on utilise de préférence le couplage des azotures sans racémisation ou le procédé au dicyclohexyl carbodiimide/1-hydroxy-benzotriazole dit ci-après DCC/HOBt ou DCC/3-hydroxy-4-oxo-3,4-dihydro-1,2,3-benzotriazine (OOBt). On peut également utiliser les esters activés des fragments.

Pour la condensation progressive des acides aminés selon b), on utilise de préférence les esters activés des acides benzyloxycarbonylaminés tels que, par exemple, l'ester du N-hydroxysuccinimide ou l'ester 2,4,5-trichlorophénylique et l'ester 4-nitrophénylique. L'aminolyse des deux derniers esters activés est très bien catalysée par des composés N-hydroxylés qui ont à peu près l'acidité de l'acide acétique, par exemple, le 1-hydroxybenzotriazole.

Comme groupes protecteurs intermédiaires des groupes amino, on emploie des groupes usuels tels que t.butoxycarbonyle (Boc), benzyloxycarbonyle (Z), isobornyloxycarbonyle (IBOC) etc...

Les groupes carboxyliques peuvent également être protégés, si nécessaire, par estérification (par exemple esters méthylique, éthylique, benzylique, etc...).

La réaction de condensation peut être effectuée en présence d'un solvant. Le solvant peut être choisi parmi ceux qui sont connus pour être utilisables dans les réactions de condensation de peptides. Ainsi, on peut mentionner à titre d'exemples les solvants anhydres ou aqueux ci-après : diméthylformamide, diméthylsulfoxyde, pyridine, chloroforme, dioxane, dichlorométhane, tétrahydrofurane, ainsi que des mélanges appropriés de tels solvants.

La température réactionnelle est choisie dans la gamme connue pour les réactions conduisant à la formation des liaisons peptides, par exemple normalement dans la gamme d'environ -20 °C à environ 30 °C. De plus, les matières précurseurs (peptides protégés) des composés recherchés selon la présente invention peuvent également être facilement préparés par des procédés de synthèse en phase solide.

Après la fin de la réaction de condensation recherchée, si le produit porte des groupes protecteurs, ils peuvent être éliminés par des procédés habituels. Parmi de tels procédés habituels, on peut citer : la réduction catalytique en présence d'un catalyseur tel que le noir de palladium, le carbone sur palladium, le platine, etc..., la solvolyse au moyen d'acide fluorhydrique, d'acide trifluoroacétique, etc...et la réduction avec le sodium métallique dans l'ammoniac liquide.

On utilise notamment l'acide trifluoroacétique (TFA) pour éliminer les groupes Boc (amino protecteurs) et une saponification pour éliminer les groupes esters protecteurs des groupes carboxyliques.

Le peptide ainsi produit à l'issue de l'étape a) ou b) peut être récupéré du mélange de produits réactionnels par des processus connus pour la récupération des peptides, par exemple par extraction, distribution, chromatographie sur colonne, etc... Le peptide peut également être

récupéré sous forme d'un sel ou d'un composé complexe métallique.

Le peptide préparé ci-dessus est alors condensé selon des techniques classiques de la chimie des peptides avec une amine de formule $H_2N - C_nH_{2n}Y$ (II), sur la partie carboxylique terminale de ce dernier suivant le schéma réactionnel.

$$\text{Boc. Tyr - D Ala - Gly - A -B-OH} + H_2N - C_nH_{2n}Y$$
(I)

L'amine de formule (II) est préparée par des techniques classiques.

Par exemple, pour préparer l'amine de formule II, dans laquelle le radical Y qui est un groupe sulfonamido, $-NHSO_2X$, on fait réagir un sulfochlorure de formule $XSO_2Cl$ sur une diamine de formule $H_2N-(CH_2)_n-CH_2$, en excès par rapport au sulfochlorure pour obtenir une monosubstitution selon le schéma réactionnel.

$$XSO_2Cl + H_2N-(CH_2)_n-NH_2 \longrightarrow H_2N-(CH_2)_n-NHSO_2X$$

La préparation de l'amine ci-dessus est notamment illustrée par P. STENBERG et al. (1971) Acta Pharmaceutica Suecica, 8, 497.

Pour préparer l'amine de formule II, dans laquelle le radical Y est un groupe -NH COX, on fait réagir un chlorure d'acide de formule YCOCl sur une diamine protégée sur l'une de ses fonctions amine par un radical benzyloxycarbonyle (Z) suivant le schéma réactionnel.

$$H_2N-(CH_2)_n-NH_2 + ZCl \longrightarrow Z-NH-(CH_2)_n-NH_2$$

$$Z-NH-(CH_2)_n-NH_2 \xrightarrow{XCOCl} Z-NH-(CH_2)_n-NH-COX \xrightarrow[AcOH]{HBr}$$

$$H_2N-(CH_2)_n-NHCOX, HBr$$

La préparation décrite ci-dessus est notamment illustrée par D.D. CLARKE et al. (1959) Arch. of Bioch. and

0005658



Biophys., 79, 338.

Selon un mode de réalisation particulier du procédé de l'invention, les peptides de formule I sont préparés en phase liquide de la façon suivante :

1 - Préparation du fragment Tyr - D.Ala

2 - Préparation du fragment Gly - L Phe - Met

3 - Condensation des fragments 1 et 2

4 - Condensation du fragment 3 avec l'amine de formule II

$$H_2N - C_n H_{2n} - Y$$

Cette préparation est illustrée sur le schéma ci-après.

Les articles de la littérature indiqués ci-dessous sont donnés à titre d'illustration des procédés usuels de préparation des peptides.


1. C. GARBAY-JAUREGUIBERRY, B.P. ROQUES, R. OBERLIN, M. ANTEUNIS, et A.K. LALA B.B.R.C., 71, 558-565, 1976.
2. C. GARBAY-JAUREGUIBERRY, B.P. ROQUES, R. OBERLIN, M. AUTEUNIS , S. COMBRISSON et J.Y. LALLEMAND FEBS Letters, 76, 93-98, 1977.
3. J.D. BOWER et al, J. Chem. Soc. Perkin Trans I, 2488-92.
4. W. WOELTER et al, Augen. Chem., Int. Ed. Engl. 15, 297 (1976).
5. E. PIETRZIK et al., Liebigs. Ann. Chem. p. 609, (1977).

**1**

(Boc - Tyr + D.Ala - OMe, HCl

↓ condensation par D.D.C. par exemple

(Boc - Tyr - D.Ala - OMe

↓ Elimination du groupe ester protecteur par D.C.C. par exemple

(Boc - Tyr - D.Ala

**2**

(Boc - Gly - L.Phe + L.Met-$OCH_3$, HCl

↓ condensation par D.C.C., H OBt par exemple

(Boc - Gly - L.Phe - Met - $OCH_3$

↓ Elimination du groupe amino protecteur (T F A)

(Gly - L Phe - Met - $OCH_3$

↓ Condensation (par ex.DCC / HOBt)

**3**

( Boc - Tyr - D.Ala - Gly - L.Phe - Met - $OCH_3$

↓ Elimination d'un groupe ester protecteur par NaOH par exemple

( Boc - Tyr - D.Ala - Gly - L.Phe - Met - OH $H_2N - C_nH_{2n}$ - Y (II)

↓ Condensation (DCC / HOBt)

**4**

Boc - Tyr - D.Ala - Gly - L.Phe - Met-NH-$C_nH_{2n}$ - Y

↓ Elimination du groupe amino protecteur par T F A

Tyr - D.Ala - Gly - L.Phe - Met - NH - $C_nH_{2n}$ - Y

Pour la préparation des peptides possédant les restes Pro ou Leu en position 5, à la place du résidu Met, on introduit ces amino acides lors de l'étape 2 de condensation Boc - Gly - L.Phe + Pro - $OCH_3$ ou Leu - $OCH_3$.

La synthèse des peptides possédant en position 4 le reste p. fluoro-phénylalanine à la place du résidu L. Phe est réalisée en préparant le fragment Boc - Gly - (D.L.) p.F - Phe-OH utilisé initialement dans l'étape 2 de la façon suivante :

(DL)p. F - Phe

MeOH,HCl

(DL)p. F - Phe - $OCH_3$, HCl            Boc - Gly

Condensation (DCC/HOBt)

Boc - Gly -(DL)p. F - Phe - $OCH_3$

Elimination du groupe ester protecteur par NaOH

Boc - Gly -(DL)p. F - Phe - OH

Les exemples suivants sont donnés à titre d'illustration du procédé de l'invention.

Dans ces exemples, la synthèse du peptide de formule

Tyr - D Ala - Gly - A - B est réalisée selon les méthodes usuelles exposées ci-dessus, la condensation finale avec l'amine de formule (II) étant seule illustrée.

<u>Exemple 1</u> - Préparation de Tyr-D Ala- $Gly-Phe-Leu-NH-\underset{CH_3}{CH}-CH_2F$, dérivé 1.

<u>Etape a</u> -

On réalise la condensation avec l'amine II suivant le schéma :

(III) (II)

$$\text{Boc-Tyr-D Ala-Gly-Phe-Leu-OH} + H_2N\text{-}CH_2 - \underset{\displaystyle CH_3}{\overset{}{CH}}\text{-}CH_2\ F \xrightarrow{DCC}$$

(IV)

$$\text{Boc-Tyr-D Ala-Gly-Phe-Leu-}\ NH\text{-}CH_2 - \underset{\displaystyle CH_3}{CH} - CH_2 - F$$

On ajoute une solution de 47 mg de (II) (0,52 mM) à une solution de 350 mg de (I) (0,52 mM) dans 5 ml de tétrahydrofurane (T H F), sous agitation, puis à 0° - 5°C une solution de 107 mg de DCC (0,52 mM) dans 3 ml de $CHCl_3$ anhydre.

On laisse sous agitation pendant 12 heures à température ambiante. Après 40 minutes de repos, un précipité de dicyclohexyl-urée DCU apparaît. A la fin de la réaction, la DCU formée est essorée, le filtrat amené à siccité sous vide. Le résidu est repris par un mélange AcOEt/$H_2O$, 50/50. La phase organique est décantée, lavée avec une solution saturée de NaCl et séchée sur $Na_2SO_4$. L'évaporation sous vide donne 232 mg (Rdt = 69 %) d'un solide amorphe blanc crème.

Formule brute : $C_{38}\ H_{55}\ O_8\ N_6\ F$

Structure confirmée par RMN, UV.

Etape b - déprotection

Préparation du trifluoroacétate (TFA) du peptide IV obtenu ci-dessus

$$\text{Boc-Tyr-D Ala-Gly-Phe-Leu-NH-}\underset{\displaystyle CH_3}{CH} - CH_2 - F\ (IV) \xrightarrow{TFA}$$

$$\text{TFA de Tyr-D Ala-Gly-Phe-Leu-NH-}CH_2\text{-}\underset{\displaystyle CH_3}{CH} - CH_2\ F \quad (I)$$

On dissout 200 mg du composé IV dans 1 ml de TFA, on laisse réagir pendant 10 mn et on évapore sous vide anhydre. Le résidu sirupeux est dissous dans le minimum de

$CH_2Cl_2$ anhydre. La solution est versée dans 20 ml d'éther ($Et_2O$) anhydre. Le précipité de peptide I formé est filtré, lavé à pH $\simeq$ 4, séché sous vide anhydre (Poids 181 mg - Rdt = 90 %) Rf ($BuOH-AcOH-H_2O$ ; 4-1-1) = 0,8

Formule brute : $C_{33}$ $H_{47}$ $O_6$ $N_6$ F, TFA = 755

Point de fusion : 203°.

Exemple 2 -

Préparation de Tyr-D Ala-Gly-Phe-Leu-NH-$CH_2$ $CH_3$, dérivé 2.

Etape a -

On réalise la condensation du peptide III avec l'amine II suivant le schéma

(III) (II)

Boc-Tyr-D Ala-Gly-Phe-Leu- OH + $H_2N$ - $CH_2$-$CH_2$ - $CF_3$ $\xrightarrow{DCC}$

(IV)

Boc-Tyr-D Ala-Gly-Phe-Leu- HN - $CH_2$-$CH_2$ - $CF_3$

On ajoute une solution de 60 mg (0,52 mole) de l'amine II dans 5 ml de THF, à une solution de 350 mg (0,52 m mole) du peptide III dans 5 ml de THF, sous agitation, puis à 0° -5 °C une solution de 107 mg (0,52 mmole) de DCC dans 3 ml de $CHCl_3$ anhydre. On laisse sous agitation pendant 12 h à température ambiante. Après un repos de 40 mn, un précipité de DCU apparaît. A la fin de la réaction, la DCU formée est essorée; le filtrat amené à siccité sous vide. Le résidu est repris par un mélange d'acétate d'éthyle-eau (AcOET/$H_2O$,50/50). La phase organique est décantée, lavée par une solution de $NaHCO_3$ puis de NaCl saturée et séchée sur $Na_2SO_4$. L'évaporation sous vide donne 238 mg (Rdt = 60 %) d'un solide amorphe jaune ocre.

Point de fusion : F = 168 °; Rf = 0,50 ($BuOH-AcOH-H_2O$, 4-1-1).

Formule brute : $C_{37}$ $H_{49}$-$O_7$ $N_5$ $F_3$

Structure confirmée par RMN, UV.

Etape b -

Préparation de trifluoroacétate du peptide IV obtenu ci-dessus

Boc-Tyr-D Ala-Gly-Phe-Leu-NH - $CH_2$-$CH_2$ - $CF_3$ (IV) $\xrightarrow{TFA}$
TFA de Tyr-D Ala-Gly-Phe-Leu-NH - $CH_2$-$CH_2$ - $CF_3$ (I)

On dissout 200 mg (0,26 m mole) de peptide IV dans 1 ml de TFA et on laisse en contact pendant 10 mn, puis on évapore sous vide anhydre. Le résidu sirupeux est dissous dans le minimum de $CH_2Cl_2$ anhydre. La solution est versée dans 20 ml de $Et_2O$ anhydre. Le précipité de composé I formé est filtré, lavé (à pH $\simeq$ 4), séché sous vide anhydre pour obtenir 182 mg de composé I (Rdt = 90 %)
Rf (BuOH-AcOH-$H_2O$ ; 4-1-1) = 0,40.
Formule brute : $C_{32}H_{41}$ $O_5$ $N_6F_3$. TFA = 777
Point de fusion : 185 °C.

Exemple 3 -
Préparation de Tyr-DAla- Gly-Phe-Pro-NH($CH_2$)$_2$-CH 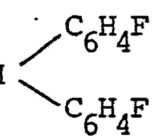 , dérivé 3 -

Etape a -

On réalise la condensation du peptide III sur l'amine II d'après le schéma ci-dessous

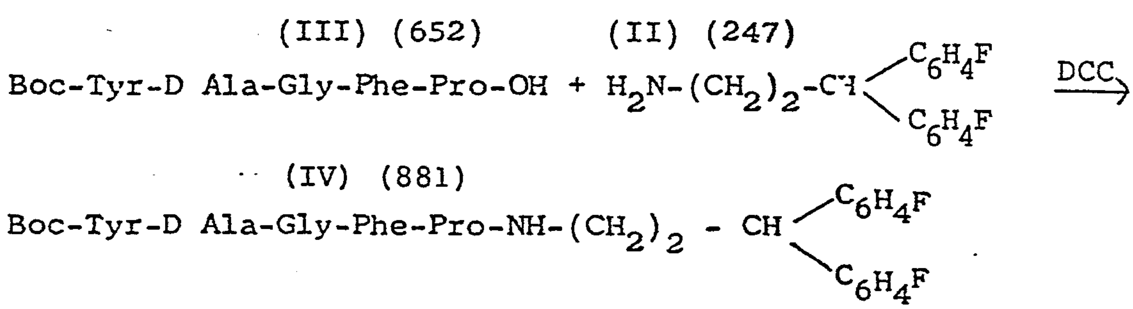

(III) (652) (II) (247)
(IV) (881)

On ajoute lentement sous agitation, à 0° - 5°C, une solution de 200 mg de DCC dans 10 ml de $CH_2Cl_2$ anhydre, à un mélange de 652 mg du peptide III (1 mM) dans 10 ml de THF anhydre et 247 mg de l'amine II dans 5 ml d'$Et_2O$.

(Cette solution est obtenue par addition de 1 équivalent de NaOH à 284 mg de chlorhydrate d'amine II dans un mélange de 5 ml d'$Et_2O$ et 5 ml d'$H_2O$).

On laisse sous agitation pendant 24 h. On essore la DCU formée, on lave avec $CH_2Cl_2$, évapore à siccité, reprend par AcOEt, lave la phase organique par une solution de $HNaCO_3$ puis de NaCl et on sèche sur $Na_2SOCl$ pour obtenir 475 mg (Rdt : 54 %) d'une poudre jaune paille.

Après recristallisation dans $Et_2O$,F = 130 °C, Rf = 0,28 (BuOH-AcOH-$H_2O$ ; 4-1-1).

<u>Etape b</u> - Déprotection

Préparation du chlorhydrate du peptide IV obtenu ci-dessus :

Boc-Tyr-D Ala-Gly-Phe-Pro-NH-$(CH_2)_2$ - 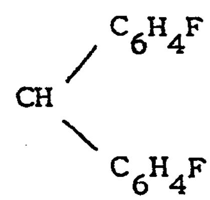 (IV)(881)

Tyr-D Ala-Gly-Phe-Pro-NH $(CH_2)_2$ - 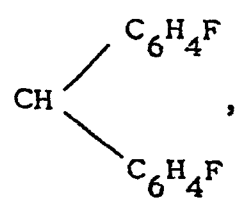 ,HCl(I)

(817,5)

On dissout 400 mg du peptide IV (0,454 m mole) dans 3 ml de dioxanne anhydre et déperoxydé, on filtre un léger insoluble et on ajoute 4 ml de HCl dans du dioxanne (24 % en poids). On laisse en contact pendant 1 h, puis on évapore sous vide anhydre et on recouvre d'$Et_2O$ anhydre. On délite la poudre obtenue, la filtre, la lave avec $Et_2O$ jusqu'à pH ≃ 4. On sèche sous vide anhydre pour obtenir 340 mg (Rdt ∼ 90 %) du composé I. Rf (BuOH-AcOH-$H_2O$ ; 4-1-1) = 0,25

Formule brute : $C_{41}$ $H_3$ $O_7$ $N_5$ $F_2$, HCl = 817,5.

<u>Exemple 4</u> -

Préparation de Tyr-D Ala- Gly-Phe-Met-NH-$CH_2$-$CH_2$- 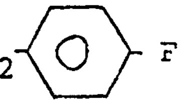 ,

dérivé 4.

Etape a -

On réalise la condensation du peptide III sur l'amine II d'après le schéma réactionnel ci-après

(III) (II)

Boc-Try-D Ala-Gly-Phe-Met-OH + $H_2N-CH_2-CH_2$ - 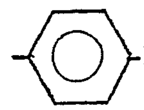 -F DCC

(IV)

Boc-Tyr-D Ala-Gly-Phe-Met-NH-$CH_2$-$CH_2$ - 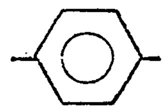 - F

On ajoute lentement sous agitation , à 0° -5°C, une solution de 200 mg de DCC dans 10 ml de $CH_2Cl_2$ anhydre, à un mélange de 600 mg de peptide III dans 10 ml de THF anhydre et 130 mg de l'amine II dans 5 ml d'$Et_2O$. (Cette solution est obtenue par addition de l'équivalent de NaOH à 145 mg de chlorhydrate d'amine II dans un mélange de 5 ml d'$H_2O$ et de 5 ml d'$Et_2O$).

On laisse sous agitation pendant 24 h. On essore la DCU formée, la lave avec $CH_2Cl_2$, évapore à siccité, reprend par AcOEt, lave la phase organique par une solution de $HNaCO_3$ puis de NaCl. On sèche sur $Na_2SOCl$ pour obtenir 660 mg (Rdt = 92 %). Poudre jaune pâle.

Après recristallisation dans $Et_2O$, F = 108 °C. Rf = 0,18 (BuOH-AcOH-$H_2O$ ; 4-1-1).

Analyse du peptide IV pour $C_{41}H_{53}N_6O_8SF$

Calculée : C % = 60,9 H % = 6,6 N % = 10,4 F % = 2,3

Trouvée : C % = 61,2 H % = 6,5 N % = 10,3 F % = 2,5

Etape b - Déprotection par HCl-dioxanne -

Elle est réalisée comme à l'exemple 3.

Rdt = 95 % ; F = 186-190 °C; Rf = 0,31 (BuOH-AcOH-$H_2O$ ; 4-1-1)

Analyse pour : Tyr-D Ala-Gly-Phe-Met-NH-$CH_2$-$CH_2$ - 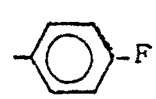 -F(I)

$C_{36} H_{46} N_6 O_6$ SF Cl

Calculée : C % = 58,03 H % = 6,18 N % = 11,30 Cl %= 4,8

Trouvée : C % = 58,10 H % = 6,4 N % = 10,9 Cl %= 5,1

Exemple 5 -

Préparation de Tyr-D Ala- Gly-Phe-Met-NH-$(CH_2)_2$-NH-$CH_2$-$CF_3$,

Dérivé 5.

Etape a -

III (687) (II) (142) DCC

Boc-Tyr-D Ala-Gly-Phe-Met-OH + $H_2N(CH_2)_2$-NH-$CH_2$-$CF_3$ ⟶

(IV) (811)

Boc-Tyr-D Ala-Gly-Phe-Met-NH $(CH_2)_2$-NH-$CH_2$-$CF_3$

On ajoute lentement sous agitation à 5° environ, une solution de 120 mg de DCC dans 10 ml de $CH_2Cl_2$ anhydre, à un mélange de 482 mg de peptide III (0,7 mM) et 100 mg d'amine II dans 15 ml de THF anhydre. On agite 24 h et on essore la DCU formée, on lave avec $CH_2Cl_2$, on évapore et on reprend par AcOEt ; on lave la phase organique par $NaHCO_3$, puis NaCl saturé. On sèche et on évapore sous vide. On obtient un résidu blanc de peptide IV ; poids 408 mg, Rf = 0,38 (BuOH-ACOH-$H_2O$, 4-1-1). On recristallise dans AcOEt-MeOH (50/50).

Poids : 360 mg. Cristaux blancs. F = 127 °C -Rdt.≃ 72 %.

Formule brute : $C_{37} H_{47} O_8 N_5 SF_3$ = 811.

Etape b : Déprotection

Préparation du trifluoroacétate du peptide IV obtenu ci-dessus :

(IV) (811)

Boc-Tyr-D Ala-Gly-Phe-Met-NH-$(CH_2)_2$-NH-$CH_2$-$CF_3$ —TFA⟶

Tyr-D Ala-Gly-Phe-Met-NH-$(CH_2)_2$-NH-$CH_2$-$CF_3$, TFA (I)

(824)

On dissout 400 mg du peptide IV dans 2 ml de TFA, on maintient en contact pendant 10 mn, et on évapore sous vide anhydre. Le résidu sirupeux est dissous dans le mini-

mum de $CH_2Cl_2$ anhydre. La solution est versée dans 20 ml de $Et_2O$ anhydre. Le précipité formé est filtré, lavé (PH ≃ 4), séché sous vide anhydre. P = 316 mg, Rdt = 91 %, Rf ($BuOH-AcOH-H_2O$ ; 4-1-1) = 0,42.

Formule brute : $C_{32}H_{39}O_6N_5SF_3$ , TFA = 824

Exemples 6 à 11 -

Préparation de Tyr-D Ala- Gly-pF Phe-Met-NH-$(CH_2)_2$-NH-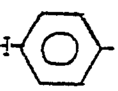-F

Etape a -

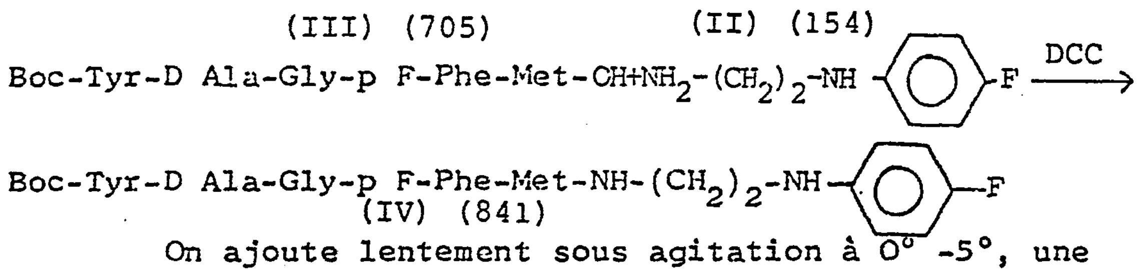


On ajoute lentement sous agitation à 0° -5°, une solution de 135 mg de DCC dans 10 ml de $CH_2Cl_2$ anhydre, à un mélange de 400 mg de peptide III (0,56 mM) dans 10 ml de THF anhydre et 86 mg d'amine II (0,56 mM) dans 5 ml d'$Et_2O$. (Cette solution est obtenue par additionde l'équivalent de NaOH à 190,5 mg de chlorhydrate de II dans un mélange de 5 ml d'$Et_2O$ et 5 ml d'$H_2O$).

On laisse sous agitation pendant 24 h. On essore la DCU formée, lave avec ($CH_2Cl_2$), évapore à sec, reprend par AcOEt, lave la phase organique par une solution de $HNaCO_3$ puis de NaCl, et on sèche sur $Na_2SOCl$ pour obtenir 306 mg (Rdt : 65 %) d'une poudre jaune pâle.

Après recristallisation dans $Et_2O$. F = 128 °C

Rf = 0,28 ($BuOH-AcOH-H_2O$ ; 4-1-1).

Formule brute : $C_{41}H_{53}O_8N_7SF_2$

Etape b - Déprotection

Préparation du chlorhydrate du peptide IV obtenu ci-dessus

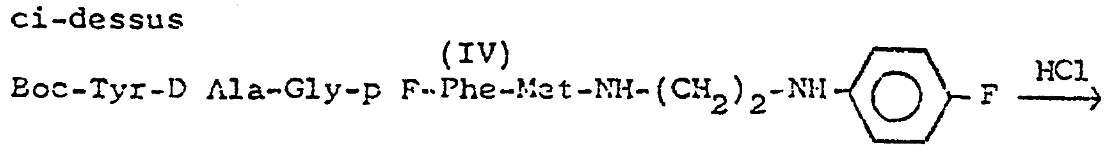

Tyr-D Ala-Gly-p Fluoro-Phe-Met-NH-$(CH_2)_2$-NH-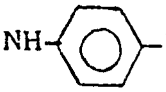-F, HCl.(I)

Chlorhydrate : On dissout 300 mg de peptide (IV) dans 3 ml de dioxanne anhydre et déperoxydé, on filtre un léger insoluble, et on ajoute 3 ml de HCl dans du dioxanne à 24% en poids. On laisse en contact pendant 1 h, puis on évapore sous vide anhydre. On recouvre d'$Et_2O$ anhydre, On délite la poudre obtenue, filtre, lave par $Et_2O$ jusqu'à pH $\simeq$ 4. On sèche sous vide anhydre (P = 302 mg, Rdt 98 %).

Rf (BuOH-AcOH-$H_2O$ ; 4-1-1) = 0,41

Formule : $C_{36} H_{45} N_7 S F_2$ ; HCl = 777,5
Point de fusion : 185 °

On a préparé de la même façon les composés qui figurent dans le tableau I :

TABLEAU I

| Dérivé Nº | $C_nH_{2n}$ | X | Formule brute | P.M. | F-ºC |
|---|---|---|---|---|---|
| 6 | $(CH_2)_2$ | 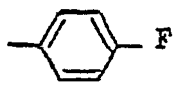 | $C_{36}H_{45}N_7SF_2$, HCl | 777,5 | 185º |
| 7 | $(CH_2)_2$ | $-CH_2$ 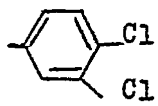 | $C_{37}H_{46}N_7O_6S$ $Cl_2F$, TFA | 919 | 182º |
| 8 | $CH_2$ | $-CH_2-CH_2-F$ | $C_{31}H_{43}O_6N_7S$ $F_2$, HCl | 715,5 | 204º-206º |
| 9 | $(CH_2)_2$ | 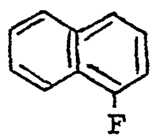 | $C_{40}H_{47}N_7O_6S$ $F_2$, HCl | 827,5 | 193º |
| 10 | $(CH_2)_2$ | 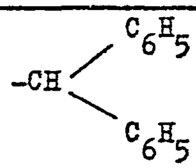 | $C_{43}H_{52}N_7O_6$ SF, TFA | 926 | 175º |
| 11 | $-CH(CH_3)-CH_2$ | 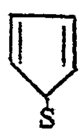 | $C_{35}H_{46}N_7S_2F$, TFA | 856 | 158º |

Exemples 12 à 19 -

Préparation de Tyr-D Ala- Gly-Phe-Met-NH-$(CH_2)_2$-NH-$SO_2$ -X,

dans lequel X est un groupe 5 diméthylamino-α-naphtyle

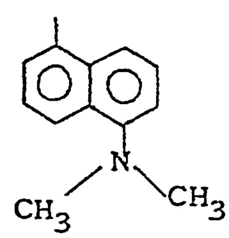

dérivé 12.

Etape a -

On opère suivant le schéma réactionnel

(III) (II)

Boc-Tyr-D Ala-Gly-Phe-Met-OH + $H_2N-(CH_2)_2-NH-SO_2-X$ ⟶

(IV)

Boc-Tyr-D Ala-Gly-Phe-Met-$NH(CH_2)_2-NH-SO_2-X$.

On ajoute une solution de 153 mg (0,52 m mole) d'amine II (F = 151 °) dans 5 ml de THF, à une solution de 355 mg (0,52 mole) de peptide IV (F = 142,°) dans 5 ml de THF, sous agitation; puis à 0-5° une solution de 107 mg (0,52 m mole) de DCC dans 3 ml de $CHCl_3$ anhydre. On laisse sous agitation pendant 12 h à la température ambiante. Après 40 mn, un précipité de DCU apparaît. A la fin de la réaction, la DCU formée est essorée ; le filtrat amené à sec sous vide. Le résidu est repris par un mélange AcOEt/$H_2O$, 50/50. La phase organique est décantée, lavée par une solution de $NaHCO_3$, puis de NACl saturée , et séchée sur $Na_2SO_4$. L'évaporation sous vide donne 386 mg (Rdt = 77 %, F = 160 - 170 °C) d'un solide amorphe jaune paille.

Rf = 0,30 (BuOH-AcOH-$H_2O$, 4-1-1).

Analyse pour $C_{47}H_{62}N_8O_{10}S_2$ :

| | | | | |
|---|---|---|---|---|
| Calculée : | C % 59 | H % 6,4 | N % 11,6 | S % 6,6 |
| Trouvée : | C % 58,91 | H % 7,02 | N % 6,8 | S % 6,15. |

Structure confirmée par RMN, UV.

Etape b-

Préparation des chlorhydrate ou trifluoroacétate de peptide IV -

a) Chlorhydrate - On dissout 100 mg de peptide IV (0,104 m mole) dans 1 ml de dioxanne anhydre et déperoxydé, filtre d'un léger insoluble, ajoute 1 ml de HCl dans du dioxanne (24 % en poids). On laisse en contact pendant 1 h, puis on évapore sous vide anhydre. On recouvre d'$Et_2O$ anhydre, délite la poudre obtenue, filtre, lave par $Et_2O$ jusqu'à pH ≃ 4. On sèche sous vide anhydre (P = 92 mg, Rdt ~ 100%)

- Rf ($BuOH-AcOH-H_2O$ ; 4-1-1) = 0,38.

Analyse pour $C_{42}H_{54}N_8O_8S_2$ . HCl :

Calculée : C % = 56,2  H % = 6,0  N % = 12,5  Cl % =3,95

Trouvée : C % = 56,4  H % = 5,2  N % = 11,7  Cl % =4,10

b) Trifluoroacétate -

On dissout 100 mg (0,104 m mole) de peptide IV dans 0,5 ml de TFA et on laisse en contact 10 mn, puis on évapore sous vide anhydre. Le résidu sirupeux est dissous dans le minimum de $CH_2Cl_2$ anhydre. La solution est versée dans 10 ml de $Et_2O$ anhydre. Le précipité formé est filtré, lavé (pH≃4), séché sous vide anhydre (P = 85 mg, Rdt = 85 %).

Rf ($BuOH-AcOH-H_2O$ ; 4-1-1) = 0,36

On a préparé de la même façon les composés de formule I du Tableau II ci-dessous.

TABLEAU II - Y = $-NHSO_2X$

| Nº | n | X | Formule brute | P.M. | F- ºC |
|---|---|---|---|---|---|
| 12 | 2 | 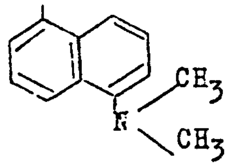 | $C_{42}H_{54}N_8O_8S_2$, HCl | 898,50 | 174º |
| 13 | 5 | 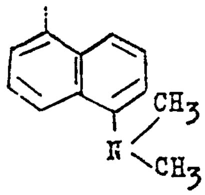 | $C_{45}H_{60}O_8N_8S_2$, HCl | 940,5 | 187º |
| 14 | 2 | 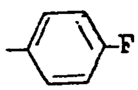 | $C_{36}H_{46}O_8N_7S_2F$, HCl | 823,5 | 187º |
| 15 | 2 | 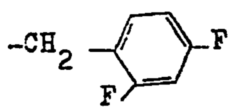 | $C_{37}H_{47}O_8N_7S_2F_2$, HCl | 855,5 | 142º |
| 16 | 1 | $-CH_2-CH_2-F$ | $C_{31}H_{44}O_8N_7S_2$, TFA | 838 | 159º |
| 17 | 3 | 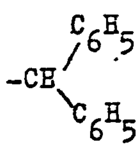 | $C_{44}H_{55}O_8N_7S_2$, TFA | 986 | 159º |
| 18 | 2 | 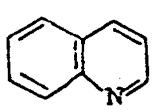 | $C_{39}H_{48}O_8N_8S_2$, HCl | 856,5 | 194º |
| 19 | 2 | 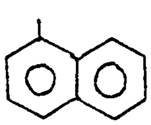 | $C_{43}H_{54}O_8N_7S_2$, HCl | 896,5 | 197º |

Exemples 20 à 25 -

Préparation de Tyr-D Ala- Gly-Phe-Met-NH-$(CH_2)_2$- 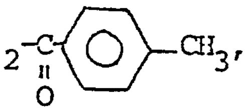

dérivé 20.

Etape a

On opère suivant le schéma réactionnel

(III) (687) (II) (217)

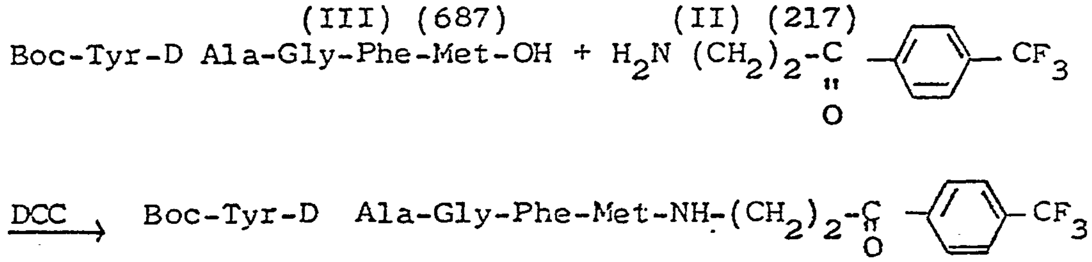


(IV) (786)

On ajoute une solution de 113 mg (0,52 m mole) d'amine II (F = 151°) dans 5 ml de THF, à une solution de 357 mg (0,52 m mole) de peptide (III) (F = 142°) dans 5 ml de THF, sous agitation ; puis à 0-5° une solution de 107 mg (0,52 m mole) de DCC dans 3 ml de $CHCl_3$ anhydre. On laisse sous agitation pendant 12 h à température ambiante. Après 40 mn, un précipité de DCU apparaît. A la fin de la réaction, la DCU formée est essorée, le filtrat amené à sec sous vide. Le résidu est repris par un mélange AcOEt/$H_2O$, 50/50. La phase organique est décantée, lavée par une solution de $NaHCO_3$ puis de NaCl saturées et séchée sur $Na_2SO_4$. L'évaporation sous vide conduit à un solide amorphe jaune paille ( P = 314 mg; Rdt = 77 %, F = 168 °C. Rf = 0,32 (BuOH-AcOH-$H_2O$, 4-1-1)

Formule brute : $C_{43}$ $H_{51}$ $O_8$ $N_6$ S $F_3$

Structure confirmée par RMN, UV.

Etape b Déprotection

Préparation de trifluoroacétate de peptide IV -

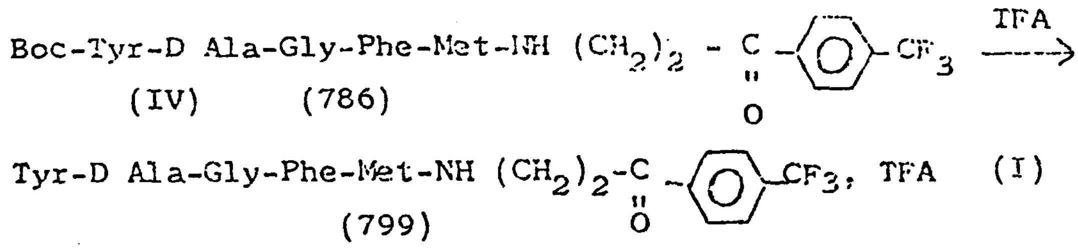


(IV) (786)

(799) (I)

On dissout 300 mg (0,382 m mole) de peptide IV dans 1,5 ml de TFA, et on maintient en contact pendant 10 mn, évapore sous vide anhydre. Le résidu sirupeux est dissous dans le minimum de $CH_2Cl_2$ anhydre. La solution est versée dans 30 ml de $Et_2O$ anhydre. Le précipité formé est filtré, lavé (pH ≃ 4), séché sous vide anhydre (P = 258 mg, Rdt = 85 %).

Rf (BuOH-AcOH-$H_2O$ ; 4-1-1) = 0,31.

Formule brute : $C_{38}H_{45}O_6N_6SF_3$, TFA = 799.

On a préparé de la façon suivante les composés de formule générale I.

L Tyr-D Ala-L Gly-Phe-Met-NH- $(CH_2)_n$ - CO - X

donnés au Tableau III ci-dessous :

TABLEAU III

| N° | n | X | Formule brute | PM | F-°C |
|---|---|---|---|---|---|
| 20 | 2 | 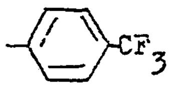 | $C_{38} H_{45} O_7 N_6 S F_3$,TFA | 799 | 168° |
| 21 | 2 | 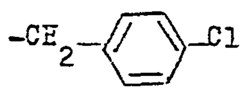 | $C_{37} H_{44} O_7 N_6$ S F Cl,HCl | 807 | 178° |
| 22 | 4 | $CH_2 - CH_2 - F$ | $C_{35} H_{49} O_7 N_6$ S F, HCl | 752,5 | 181° |
| 23 | 2 | 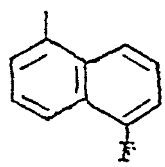 | $C_{41} H_{47} O_7 N_6$ S F, TFA | 899 | 202° |
| 24 | 2 | 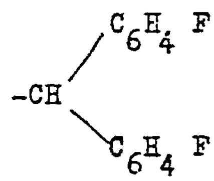 | $C_{44} H_{50} O_6 N_6$ S F, HCl | 880,5 | 197° |
| 25 | 2 | 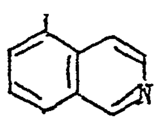 | $C_{41} H_{47} O_7 N_7$ S, TFA | 882 | 149° |

Exemples 26 à 30 -

Préparation du /1-(4-fluorobenzyl)-carboxylamino/ éthyl-D Ala$^2$- Met-enkephalinamide de formule

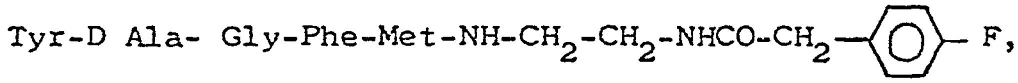

dérivé 26

Etape a-

On opère suivant le schéma réactionnel suivant :

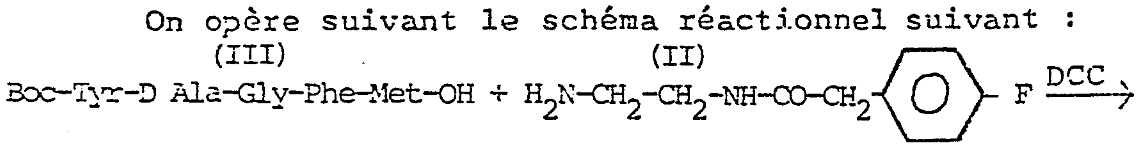

Boc-Tyr-D Ala-Gly-Phe-Met-NH-$CH_2$-$CH_2$-NH-CO-$CH_2$- 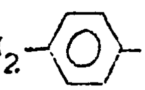 -F

(IV)

On ajoute lentement sous agitation à 0° -5°, une solution de 220 mg de DCC dans 10 ml de $CH_2Cl_2$ anhydre, à un mélange de 720 mg de peptide III (F = 142 °C) et 210 mg d'amine II dans 15 ml de THF anhydre. On agite 24 h, essore la DCU formée, lave avec $CH_2Cl_2$, évapore, reprend par AcOEt, lave la phase organique par $NaHCO_3$ puis NaCl saturé. On sèche sur $Na_2SO_4$ et évapore sous vide pour obtenir 852 mg de résidu blanc.

Rf = 0,34 (BuOH-AcOH-$H_2O$, 4-1-1).

Après recristallisation dans AcOEt-MeOH (50/50), P = 650 mg. Cristaux blancs. F = 124-126 °C. Rdt≃68 %.

Analyse pour $C_{43}$ $H_{56}$ $O_9$ $N_7$ S F

| | | | | |
|---|---|---|---|---|
| Calculée : | C % = 60 | H % = 6,5 | N % = 11,5 | F % = 2,2 |
| Trouvée : | C % = 60,7 | H % = 6,9 | N % = 10,6 | F % = 1,8 |

Etape b - Déprotection du peptide IV pour obtenir le composé de formule I ci-dessous

Tyr-D Ala-Gly-Phe-Met-NH-$(CH_2)_2$-NHCOC$H_2$ 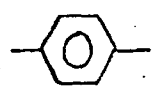 F (I)

On dissout 400 mg de peptides IV dans 5 ml de dioxanne anhydre déperoxydé, on ajoute à 0 °C, 2 ml d'une solution de dioxanne-HCl à 24 % en poids. Après une mise en contact de 1 h à 0 °C, on évapore sous vide anhydre et on recouvre de $Et_2O$, pour obtenir une poudre blanche. On filtre, lave (pH≃ 4), sèche sous vide anhydre (P= 365 mg Rdt 90 %).

Rf = 0,22 (BuOH-AcOH-$H_2O$ ; 4-1-1).

Analyse pour $C_{38}$ $H_{49}$ $O_7$ $N_7$ S F Cl

| | | | | |
|---|---|---|---|---|
| Calculée : | C % = 56,9 | H % = 6,1 | N % = 12,2 | Cl % =4,4 |
| Trouvée : | C % = 56,1 | H % = 6,5 | N % = 12,6 | Cl % =4,9 |

On a préparé de façon analogue des composés de formule générale I Tyr-D Ala-Gly-Phe-Met-NH-$(CH_2)_n$-NH-CO-$(CH_2)_m$-X, qui sont donnés au Tableau IV.

TABLEAU IV

| Nº | n | m | X | Formule brute | PM | F-ºC |
|---|---|---|---|---|---|---|
| 26 | 2 | 1 | 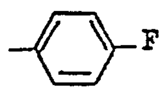 | $C_{38} H_{48} O_7 N_7 S F$, HCl | 801,5 | 151º |
| 27 | 2 | 2 | $-(CH_2)_2 - Cl$ | $C_{35} H_{50} O_7 N_7 S Cl$, TFA | 860,5 | 201º |
| 28 | 2 | 1 | 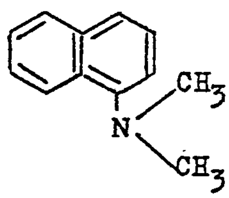 | $C_{42} H_{56} O_7 N_8 S$, TFA | 953 | 187º |
| 29 | 2 | 1 | 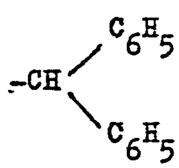 | $C_{45} H_{64} O_7 N_7 S$, TFA | 959 | 164º |
| 30 | 2 | 2 | 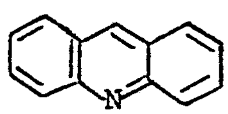 | $C_{46} H_{54} O_7 N_8 S$, HCl | 898,5 | 143º |

Exemple 31 -

Préparation du tétrapeptide Tyr-D Ala- Gly-Phe-NH-$(CH_2)_2$-NH-CO-$CH_2$-$C_6H_4$ - F

Etape a

Boc -Tyr-D Ala-Gly-Phe-OH (III) + $H_2N$ $(CH_2)_2$-NH-CO-$CH_2$-$C_6H_4$-F (II) $\xrightarrow{DCC}$

Boc-Tyr-D Ala-Gly-Phe-NH-$(CH_2)_2$-NH-CO-$CH_2$-$C_6H_4$ - F (IV)

On ajoute lentement sous agitation à 0-5 °C, 220 mg de DCC dans 10 ml de $CH_2Cl_2$ anhydre, à un mélange de 584 mg de peptide III ( 1 mM) et 210 mg d'amine II dans 15 ml de THF anhydre. On agite 24 h, on essore la DCU formée. On lave avec $CH_2Cl_2$. On évapore et on reprend dans de l'acétate d'éthyle. On lave la phase organique par une solution de bicarbonate de Na et de ClNa saturée. On sèche sur sulfate de sodium et on évapore sous vide. On obtient un résidu jaune (P = 458 mg) Rf = 0,28 ($BuOH-AcOH-H_2O$, 4-1-1). On recristallise dans un mélange acétate d'éthyle-méthanol (50/50). P = 412 mg. Cristaux jaunes pâles. F = 162° - Rdt 60 %.

Etape b - Déprotection du peptide - Préparation du chlorhydrate.

On dissout 400 mg de peptide IV dans 4 ml de dioxanne anhydre et déperoxydé, on filtre d'un léger insoluble, ajoute 4 ml de HCl dans un dioxanne à 24 % en poids. On laisse en contact pendant 1 h, puis on évapore sous vide anhydre. On recouvre d'$Et_2O$ anhydre, délite la poudreobtenue, filtre, lave par $Et_2O$ jusqu'à pH ~4. On sèche sous vide anhydre (P = 420 mg, Rdt ~ 100 %) Rf ($BuOH-AcOH-H_2O$ ; 4-1-1) = 0,20.

Les résultats des essais pharmacologiques et toxicologiques qui sont rapportés ci-après mettent en évidence les propriétés des dérivés de l'invention,notamment une faible toxicité ainsi que des activités analgésique, psychotrope et antidiarrhéique.

L'invention a donc encore pour objet un médicament présentant en particulier des activités analgésiques, psychotrope et antidiarrhéique, caractérisé en ce qu'il contient à titre de principe actif une quantité efficace d'uncomposé de formule I ou d'un sel d'addition avec un acide thérapeutiquement acceptable de ce composé.

## 1. ETUDE PHARMACOLOGIQUE

### A/ Activités analgésique, psychotrope et antidiarrhéique

I. Action analgésique par rapport à la morphine comme substance de référence qui a été contrôlée :

a) Sur organe isolé.

- iléon de cobaye

METHODE.

On a utilisé la technique de stimulation transmurale de l'iléon de cobaye de PATON (W.D.M. PATON, "The response of the guinea-pig ileum to electrical stimulation by coaxial electrodes" - J. Physiol. (London), 127, 40P-41P (1955) et PATON (W.D.M.) - Brit.J. Pharmacol. 11).

- Les essais ont été réalisés dans une cuve de 35 ml irriguée par une solution de KREBS.
- Les stimulations électriques, supramaximales (6 par minute - 0,15 Hertz) étaient ajustées à chaque essai. Courant rectangulaire - durée 0,5 milliseconde.
- Les concentrations des différents produits étudiés, ainsi que celles de la morphine qui a servi de corps de référence, sont exprimées en nanomoles (nM) par litre.
- Pour chaque concentration, le temps de contact a été de 3 minutes. Chaque essai était séparé du suivant par une période de 10 à 20 minutes.
- Les résultats sont exprimés en pourcentage d'inhibition de la concentration consécutive à la stimulation électrique de l'iléon. On a ensuite calculé les doses actives 50 par la méthode de Parker et Waud (J. of pharmacology, 1971, 177, n°4) qui ont été comparées à celle de la morphine dans des essais concomitants.

RESULTATS.

Les résultats sont présentés dans le tableau V suivant.

TABLEAU 5

| Dérivé | Conc. nM/l | Nombre d'essais | % d'inhibition | Activité D.A. 50 nM/l | Activité Morphine =100 |
|---|---|---|---|---|---|
| n° 12 | 12 | 3 | 28,3 | 24,5 | 147 |
| | 18 | 3 | 35,5 | | |
| | 24 | 3 | 51,3 | (8,8-48,6) | |
| | 60 | 3 | 76,3 | | |
| n° 13 | 150 | 4 | 34,2 | | |
| | 300 | 4 | 46,4 | | |
| | 600 | 4 | 68,1 | | |
| | 900 | 1 | - | 320 | 12,7 |

b) Vas deferens de souris.

METHODE.

On a utilisé une technique de stimulation transmurale du canal différent de la souris, adaptée à celle décrite par HUGHES et KOSTERLITZ (J.HUGUES, H.W.KOSTERLITZ and Frances M.LESLIE, "Effect of morphine ou adrenergic transmission in the mouse vas deferens. Assessment of agonist and antagonist potencies of narcotic analgesics".-Br J. Pharmac. 53 371-381 (1975).

- Les essais ont été réalisés dans une cuve de 7 ml irriguée par une solution de KREBS.
- Les stimulations électriques, supramaximales (6 par minute) étaient ajustées à chaque essai.
- Les concentrations des différents produits étudiés sont exprimées en nM par litre.
- Pour chaque concentration le temps de contact a été de 3 minutes. Dans les cas où le maximum d'inhibition a été atteint en un temps plus court, le fait a été signalé. Chaque essai était séparé du suivant par une période de 10 à 20 minutes.
- Les résultats sont exprimés en pourcentage d'inhibition

de la concentration consécutive à la stimulation électrique du canal déférent. On a ensuite calculé les doses actives 50 par la méthode de Parker & Waud (J.Pharm. Exper. Therap.96-99 (1949).

RESULTATS.

Ils sont présentés dans le tableau VI suivant.

TABLEAU VI

| Dérivé | Conc.en nM/1 | Nombre d'essais | % d'inhibition | Activité DA 50nM/1 |
|---|---|---|---|---|
| n°12 | 1,2 | 2 | 41,8 | |
| | 1,8 | 2 | 50 | 1,7 |
| | 2,4 | 2 | 63 | |
| n°13 | 600 | 1 | 38 | |
| | 750 | 1 | 55 | 650 |
| | 900 | 1 | 83,3 | |
| | 1 500 | 1 | 90,5 | |

c) Sur animal entier par la technique de la plaque chaude chez la souris.

METHODE.

Plaque chauffante (JACOB et BLOZOVSKI, Arch. Int.Pharmacodyn. 122, 287-300, 1959 : 133, 296-300, 1961).

Ces essais ont été réalisés avec des souris mâles, indemmes d'organismes pathogènes spécifiques pesant de 21 à 26 g.

Les animaux sont placés sur une plaque chauffée à 65° trois fois de suite à 5 minutes d'intervalle. Le "cut off time" est de 45 secondes. On note à chaque passage les temps de lèchement et de bond.

Les paramètres utilisés sont : le temps de lèchement à la première exposition $L_1$ (réaction peu élaborée) et le temps de bond à la troisième exposition $B_3$ (réaction élaborée apparentée à un conditionnement).

Le dérivé n°12 et la morphine ont été administrés par voie I.V.

Témoins : sérum physiologique 0,1 ml/10 g

Morphine : 0,3 1 à 3 mg/kg sous le même volume.

Dérivé n° 12 : 1, 3 et 10 mg/kg sous le même volume.

Lectures : 5 minutes après traitement : lèchement

15 minutes après traitement : Ier bond

30 à 45 minutes après traitement : saut ajusté.

<u>RESULTATS</u>.

Ils sont exposés dans le tableau VII suivant :

TABLEAU VII

| Séries | Nbre d'Obs. | 5 Minutes | | 15 Minutes | | 30 Minutes | | 45 Minutes | |
|---|---|---|---|---|---|---|---|---|---|
| | | Temps de lèchements (en sec) | Nbre d'animaux ayant résisté > 45 sec. | Temps de bonds (en sec.) | Nbre d'animaux ayant résisté > 45 sec. | Temps de sauts (en sec) | Nbre d'animaux ayant résisté > 45 sec. | Temps de sauts (en sec) | Nbre d'animaux ayant résisté > 45 sec. |
| Témoin | 21 | 6,4 | 0 | 12,6 | 0 | 5 | 0 | 4,9 | 0 |
| Morphine 0,3mg/kg | 7 | 5,1 | 0 | 28,6 | 3 | 9,4 | 0 | 4 | 0 |
| Morphine 1 mg/kg | 7 | 7,2 | 0 | 27,6 | 3 | 15 | 0 | 4,6 | 0 |
| Morphine 3 mg/kg | 7 | 10,4 | 0 | 45 | 7 | 40,5 | 6 | 34,5 | 5 |
| Dérivé Nº12 1 mg/kg | 7 | 10 | 0 | 15,4 | 0 | 10,3 | 1 | 10,7 | 1 |
| Dérivé Nº12 3mg/kg | 7 | 5,10 | 0 | 11,9 | 1 | 5,7 | 0 | 3,8 | 0 |
| Dérivé Nº12 10 mg/kg | 7 | 6,3 | 0 | 27,7 | 3 | 16,5 | 0 | 7,1 | 0 |

L'activité du dérivé N° 12 à 10 mg/kg est comparable à celle de la morphine à 1 mg/kg en intensité et en durée, ce qui, en tenant compte des molarités, donne un coefficient d'activité de 35 % environ.

2. Action psychotrope

Elle a été évaluée par la recherche d'une catalepsie chez le rat.

METHODE.

Ces essais ont été réalisés avec des rats femelles indemmes d'organismes pathogènes spécifiques pesant de 200 à 220 g.

L'effet cataleptique est mesuré en suspendant le rat par ses pattes antérieures au rebord de la cage d'expérience : un rat cataleptique reste dans cette position alors qu'un animal normal l'abandonne aussitôt, voire ne la prend pas du tout.

La catalepsie est appréciée de la façon suivante:

0 : pas de catalepsie
1 : catalepsie liminaire
2 : catalepsie d'intensité moyenne
2 : catalepsie très prononcée

Les animaux sont traités par voie sous cutanée.

Les expériences ont été effectuées à l'aveugle, l'observateur ignorant les traitements.

RESULTATS.

Ils sont exprimés par rapport à la morphine et à l'Halopéridol et sont exposés dans le tableau VIII ci-dessous.

TABLEAU VIII

| Dérivé | D.A. 50/ sous cut. |
|---|---|
| n°12 | 5 mg/kg |
| Morphine | 2,5 mg/kg |
| Halopéridol | 0,20 mg/kg |

3. L'action antidiarrhéique.

Elle a été recherchée (outre l'effet sur l'iléon de cobaye isolé) par l'épreuve à l'huile de ricin chez le rat.

METHODE. (NIEMEGEERS et Coll. Arzn. - Forsch. 22, 516-518, 1972)

Ces essais ont été réalisés avec des rats mâles de 160 à 220 g. Après un jeûne de 18 heures, chaque animal a reçu par voie orale, un millilitre d'huile de ricin.

Le dérivé N° 12, la codéine (base) en suspension dans l'eau et la gomme arabique et la Morphine (chlorhydrate) en solution ont été administrés par voie orale sous un volume de 0,5 ml pour 100 grammes, une heure avant l'huilede ricin.

Les selles de chaque animal sont examinées, dans l'ignorance du traitement 1 - 3 - 5 heures après l'administration d'huile de ricin soit 2 - 4 et 6 heures après le produit antidiarrhéique et l'on dénombre les animaux présentant de la diarrhée (ou des selles mixtes).

RESULTATS.

Dans le tableau IX ci-dessous on constate que le dérivé n° 12 est un antidiarrhéique puissant ; en effet, il protège tous les animaux pendant quatre heures à la dose de 30 mg/kg.

TABLEAU IX

| Séries | Nombre de rats | Nombre d'animaux non diarrhéiques | |
|---|---|---|---|
| | | 2 heures | 4 heures |
| Témoin * | 30 | 8 | 2 |
| Dérivé N° 12 | | | |
| 3 mg/kg | 10 | 6 | 3 |
| 10 mg/kg | 10 | 10 | 6 |
| 30 mg/kg | 10 | 10 | 10 |
| Codéine base | | | |
| 3 mg/kg | 10 | 7 | 4 |
| 10 mg/kg | 20 | 19 | 14 |
| 30 mg/kg | 10 | 10 | 8 |
| Morphine (chlorhydrate) | | | |
| 1 mg/kg | 10 | 9 | 4 |
| 3 mg/kg | 10 | 9 | 5 |
| 10 mg/kg | 10 | 10 | 8 |

* Huile de ricin seule.

B/ Reconnaissance des sites récepteurs cérébraux des enképhalines.

On a déterminé le pouvoir compétiteur des dérivés de l'invention vis-à-vis des sites de liaison à haute et moyenne affinité de la leucine-enképhaline 3-H de membranes striatales de souris.

Les conditions de préparation des membranes et d'incubation ont été les mêmes que celles qui sont décrites dans Audigier et Coll. (C. Acad. Sciences plus European J. of Pharmacol.).

Des concentrations croissantes des divers composés synthétisés ont été opposées à une concentration fixe de Leucine-Enképhaline 3-H et les déplacements observés ont permis dans chaque cas de déterminer une constante d'inhibition (Ki).

On donne comme exemple, au tableau X ci-dessous les valeurs de Ki obtenues avec les composés 12 et 13.

TABLEAU X

| Dérivé | Ki (nM) |
|---|---|
| Leu-enképhaline | 5 ± 1,8 |
| n° 12 | 5 ± 2 |
| n° 13 | 21 ± 5 |

II. ETUDE TOXICOLOGIQUE

Les dérivés de formule générale I bénéficient d'une excellente tolérance et d'une faible toxicité qui a été plus particulièrement étudiée sur les dérivés N° 12 et 13, qui ont fait l'objet d'une épreuve de toxicité aiguë chez la souris.

Les D.L. 50 obtenues sont respectivement de :
725 ± 25 mg/kg par voie IV pour le dérivé n° 12.
et 892 ± 31 mg/kg " " " pour le dérivé n° 13.

Les résultats de ces études mettent en évidence la faible toxicité et les intéressantes propriétés analgésique, psychotrope et antidiarrhéique des dérivés de l'invention qui les rendent utiles en médecine humaine et vétérinaire.

Le médicament de l'invention est administrable à l'homme par la voie orale sous forme de comprimés, comprimés dragéifiés, capsules, gouttes ou sirops. Il peut aussi être présenté, pour l'administration rectale, sous forme de suppositoires et pour l'administration parentérale sous forme de soluté injectable.

Chaque dose unitaire contient avantageusement de 5 à 100 mg de principe actif, ainsi :

- pour les comprimés de 5 à 30 mg de principe actif
- pour les suppositoires de 10 à 30 mg de principe actif
- pour les ampoules injectables de 1 ml, une solution de 0,5 à 2 % de principe actif.

Les doses administrables journellement peuvent varier de 60 mg à 200 mg de principe actif environ.

Le médicament de l'invention peut être administré à l'homme pour le traitement de la douleur ou des diarrhées.

En raison de son activité psychotrope, il est également applicable au traitement des psychoses et névroses.

## R E V E N D I C A T I O N S

1. Dérivés de peptides répondant à la formule générale

Tyr - D Ala - Gly - A - B - NH - $C_nH_{2n}$ - Y,<br>
1 2 3 4 5

dans laquelle

A est Phe ou pF-Phe,

B est Met, Leu, monofluoro-Leu, Pro, monofluoro-Pro ou une simple liaison,

Y est un atome d'halogène ; un radical alkyle en $C_1$ à $C_4$ mono- ou polysubstitué par au moins un groupe choisi parmi un atome d'halogène et un groupe phényle éventuellement substitué par au moins un atome d'halogène ; un radical amino -NHX ; un radical sulfamido $-NHSO_2X$ ; un radical carbonyle -COX ; un radical acylamino $-NHCO(CH_2)_mX$, dans lesquels X est un atome d'halogène ; un radical alkyle en $C_1$ à $C_4$ mono- ou polysubstitué par un atome d'halogène ; phényle éventuellement mono-, di- ou trisubstitué par des radicaux choisis parmi un atome d'halogène, un groupe mono- ou polyhaloalkyle en $C_1$ à $C_4$, alcoxy en $C_1$ à $C_4$ ; benzhydryle éventuellement substitué par au moins un atome d'halogène; α ou β naphtyle éventuellement substitué par un atome d'halogène, un groupe alkyle en $C_1$ à $C_4$, alcoxy en $C_1$ à $C_4$ ou di-alkyl ($C_1$-$C_4$) amino ; ou un reste d'un radical mono- ou polycyclique choisi parmi le thiophène, la quinoléine, l'isoquinoléine, l'acridine et la pyridine ; m est 0 ou un nombre de 1 à 4, et

n est 0 ou un nombre de 1 à 6,

et leurs sels d'addition avec des acides pharmaceutiquement acceptables.

2 - Dérivé selon la revendication 1, caractérisé en ce qu'il répond à la formule

Tyr - D Ala - Gly - Phe - Met - NH - $(CH_2)_2$ - $NHSO_2$ 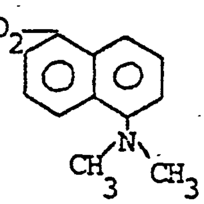

et ses sels d'addition avec des acides pharmaceutiquement acceptables.

3 - Dérivé selon la revendication 1, caractérisé en ce qu'il répond à la formule

Tyr - D Ala - Gly - Phe - Met - NH - $(CH_2)_5$ - $NHSO_2$ 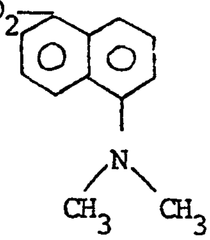

et ses sels d'addition avec des acides pharmaceutiquement acceptables.

4 - Dérivé selon la revendication 1, caractérisé en ce qu'il répond à la formule

Tyr - D Ala - Gly - Phe - NH - $(CH_2)_2$ - NHCO - $CH_2$ - $C_6H_4$ - F

5 - Procédé de préparation des dérivés de peptides de formule I, caractérisé en ce qu'on prépare des peptides

a) par une condensation fragmentaire, usuelle dans la chimie des peptides, de fragments de peptides selon le schéma de condensation 1-2 + 3-5, ou

b) par un mode de constitution progressive, dans lequel d'autres groupes fonctionnels sont bloqués intermédiairement par des groupes protecteurs éliminables par hydrolyse ou hydrogénation ou en milieu alcalin,

pour obtenir un peptide de formule III

Tyr - D Ala - Gly - A - B - OH,

dans laquelle A et B ont les définitions données à la revendication 1, et

c) on condense sur le groupe carboxylique terminal du peptide obtenu de formule III, une amine de formule II

$$H_2N - C_nH_{2n} - Y$$

dans laquelle n et Y ont les définitions données à la revendication 1.

6 - Composition thérapeutique, ayant notamment des activités analgésique, psychotrope et antidiarrhéique, caractérisée en ce qu'elle contient à titre de principe actif un composé selon l'une quelconque des revendications 1 à 4, en association avec des véhicules et excipients usuels.

7 - Composition thérapeutique, selon la revendication 6, caractérisée en ce qu'elle est présentée sous forme de doses unitaires pour l'administration orale, rectale ou parentérale, contenant de 5 à 100 mg de principe actif.

Office européen des brevets

# RAPPORT DE RECHERCHE EUROPEENNE

0005658

Numéro de la demande

EP 79 40 0268

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| X | FR - A - 2 359 817 (RECKITT)<br>* brevet entier *<br>--- | 1,5-7 | C 07 C 103/52<br>A 61 K 37/02 |
| X | FR - A - 2 347 336 (ICI)<br>* pages 1,65-69 *<br>--- | 1,5-7 | |
| | FR - A - 2 364 890 (RICHTER)<br>* pages 1-4 *<br>--- | 1,5 | |
| | FR - A - 2 365 553 (LILLY)<br>* pages 1-16 *<br>--- | 1,5 | DOMAINES TECHNIQUES RECHERCHES (Int. Cl.³)<br>C 07 C 103/52<br>A 61 K 37/02 |
| P | EP - A - 0 000 559 (WELLCOME)<br>* brevet entier *<br>--- | 1,5-7 | |
| A | UNLISTED DRUGS, Vol. 29, no. 1, janvier 1977,<br>SPECIAL LIBRARY ASSOCIATION, page 7n<br>& SCIENCE 194:330, 15 octobre 76<br>DALA<br>--------- | 1,7 | CATEGORIE DES DOCUMENTS CITES<br>X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons<br>&: membre de la même famille, document correspondant |

X Le présent rapport de recherche a été établi pour toutes les revendications

| Lieu de la recherche | Date d'achèvement de la recherche | Examinateur |
|---|---|---|
| La Haye | 24-07-1979 | RAJIC |

OEB Form 1503.1 06.78